# EUROPÄISCHE PATENTSCHRIFT

(11) **EP 2 865 355 B1**
(45) Veröffentlichungstag und Bekanntmachung des Hinweises auf die Patenterteilung: **13.09.2017**
(21) Anmeldenummer: 14188406.4
(22) Anmeldetag: 10.10.2014
(51) Int. Cl.: A61F 2/24

(54) **Verfahren und System zum Anbringen eines Implantats auf einem Katheter**
Method and system for fitting an implant to a catheter
Procédé et système de fixation d'un implant sur un cathéter

(30) Priorität: 23.10.2013 US 201361894432 P
(43) Veröffentlichungstag der Anmeldung: 29.04.2015
(73) Patentinhaber: Biotronik AG, 8180 Bülach (CH)
(72) Erfinder: Jahn, Andreas, 8046 Zürich (CH); Hofmann, Eugen, 8049 Zürich (CH); Hepke, Markus, 8006 Zürich (CH)
(74) Vertreter: Galander, Marcus

(56) Entgegenhaltungen:
- WO-A1-2014/085040
- US-A1- 2007 079 494
- US-A1- 2008 221 703
- US-A1- 2010 043 197

## Beschreibung

Die Erfindung betrifft ein Verfahren zum Anbringen einer künstlichen Herzklappe mit einem selbstexpandierenden Grundkörper auf einem Katheterschaft, wobei die Herzklappenprothese in ihrem Durchmesser durch eine Relativbewegung von mindestens zwei Bauteilen, die jeweils zumindest teilweise einen rotationssymmetrischen und sich entlang der Symmetrieachse in einer Richtung verjüngenden Raum umschließen, reduziert wird. Die Erfindung betrifft ferner ein System zur Durchführung des Verfahrens.

Unter einem intraluminalen Implantat werden im Rahmen der vorliegenden Erfindung Prothesen verstanden, die in Hohlorgane des Körpers eingebracht werden und dort verbleiben oder sich nach einer vorgegebenen Zeitspanne auflösen bzw. zersetzen (degradieren). Derartige intraluminale Implantate werden mittels eines Katheters in das jeweilige Hohlorgan eingebracht. Intraluminale Implantate haben zumeist im Wesentlichen die Form eines Röhrchens und weisen eine Gitterstruktur auf, die der Dimension des Hohlorgans entspricht, in das die Implantate eingebracht werden. Ebenfalls bekannt sind intraluminale Implantate, die künstliche Herzklappen tragen. Eine derartige künstliche Herzklappe besteht im Wesentlichen aus einer Klappe aus zumeist biologischem Gewebe, welche in einer Gitterstruktur befestigt ist, die die künstliche Herzklappe trägt. Derartige künstliche Herzklappen können ebenfalls mittels Katheter an der Stelle der natürlichen Herzklappe implantiert werden.

Die vorliegende Erfindung betrifft eine künstliche Herzklappe mit einer Gitterstruktur (im Folgenden: Herzklappenprothese), wie sie an Stelle der natürlichen Herzklappe implantiert wird..

Die Implantation einer derartigen Herzklappenprothese erfolgt über einen Katheter. Dabei wird der Katheter beispielsweise über die Leistenarterie eingeführt und die Herzklappenprothese mittels Katheter an den Implantationsort gebracht. Dort wird die Herzklappenprothese entfaltet und im Gefäß an der Position der natürlichen Herzklappe verankert. Die natürliche Herzklappe wird dabei nicht entfernt sondern lediglich von der Herzklappenprothese verdrängt.

Zum Einbringen mittels Katheter muss die Herzklappenprothese entsprechend auf dem Schaft des Katheters zum Einbringen der Herzklappenprothese angebracht werden. Dabei muss zum Einbringen der Durchmesser der Herzklappenprothese deutlich kleiner sein als am Implantationsort. Entsprechend wird die Herzklappenprothese auf den Katheterschaft komprimiert ("gecrimpt") und nach dem Einbringen am Implantationsort expandiert. Die Expansion kann dabei wie bei einem Stent selbstexpandierend oder durch Expansion eines Ballons erfolgen.

Ein Verfahren und eine Vorrichtung zum Anbringen eines Stents auf einem Katheterschaft werden in US 6,387,117 beschrieben. Nach dem dort beschriebenen Stand der Technik wird der Stent über den Katheterschaft gebracht und mittels einer Presse auf den Katheterschaft gecrimpt. Die Presse weist mindestens zwei Pressformen auf, zwischen die der Katheterschaft mit dem Stent eingebracht wird. Die Pressformen bestehen aus einer Vielzahl parallel angeordneter Plättchen mit konischen Aussparungen. Die Plättchen der beiden Formen sind dabei versetzt angeordnet. Entsprechend wird der Stent durch die jeweils benachbarten konischen Aussparungen auf den Katheterschaft gepresst.

US 2010/043197 offenbart ein Verfahren und eine Vorrrichtung zum Crimpen von selbstexpandierenden Objekten.

US 2008/221703 offenbart eine Vorrichtung zum Laden eines komprimierbaren Implantats auf einen Einführkatheter.

US 2007/079494 offenbart eine Vorrichtung zum Laden eines Stents auf einen Einführkatheter umfassend eine Vorrichtung zum Crimpen, eine Fluidquelle und ein Stützteil zum Stützen und Bewegen des Einführkatheters.

WO 2014/085040 offenbart eine Crimpwerkzeug zum Crimpen einer Herzklappenprothese.

Beim Anbringen einer Herzklappenprothese auf einem Katheterschaft nach einem Verfahren nach dem Stand der Technik besteht die Gefahr, dass die aus biologischem Gewebe bestehenden Herzklappen verkanten oder knicken. Dies würde zu einer Beschädigung der Klappen und einer daraus resultierenden Funktionsstörung führen, die unbedingt zu vermeiden ist.

Der vorliegenden Erfindung liegt daher die Aufgabe zugrunde, ein Verfahren und ein System zum Anbringen einer Herzklappenprothese derart auszugestalten, dass das Risiko einer Schädigung der Herzklappenprothese beim Anbringen auf dem Katheterschaft minimiert wird. Insbesondere soll beim Anbringen von Herzklappenprothesen ein Knicken oder Verkanten der einzelnen aus biologischem Gewebe bestehenden Herzklappen vermieden werden.

Die vorliegende Aufgabe wird verfahrensseitig durch die Merkmalskombination des unabhängigen Anspruches 1 und vorrichtungsseitig durch ein System mit den Merkmalen des unabhängigen Anspruches 5 gelöst. Vorteilhafte Ausgestaltungen der Erfindung werden in den abhängigen Ansprüchen ausgeführt.

Das erfindungsgemäße Verfahren zeichnet sich dadurch aus, dass die Relativbewegung der mindestens zwei Bauteile entlang der Längsachse der Herzklappenprothese aufeinander zu und derart ineinander erfolgt, dass sich der von den mindestens zwei Bauteilen gemeinsam umschlossene Raum verkleinert, wobei sich die Herzklappenprothese in dem von den mindestens zwei Bauteilen gemeinsam umschlossenen Raum befindet und derart orientiert ist, dass die Längsachse des Implantats und die Symmetrieachsen der rotationssymmetrischen Räume aufeinander liegen.

Beim Anbringen einer Herzklappenprothese auf dem Katheterschaft vor der Implantation muss der Durchmesser der Herzklappenprothese verkleinert werden. Dies erfordert eine Kraft, die senkrecht auf die Längsachse der zumeist rotationssymmetrischen Herzklappenprothese gerichtet ist. Im Stand der Technik wird diese Kraft durch eine Bewegung von Bauteilen erzeugt, die ebenfalls senkrecht zur Längsachse der Herzklappenprothese orientiert ist, wie die Presse aus dem vorgehend geschilderten Stand der Technik in US 6,387,117. Hier werden senkrecht zur Längsachse des Stents von oben und von unten zwei Pressenteile aufeinander zu bewegt. Über die konischen Aussparungen der Pressenteile wird die Kraft lediglich tangential am Umfang des Stents verteilt.

Die vorliegende Erfindung erzeugt die Kraft für das Anbringen und Komprimieren der Herzklappenprothese jedoch durch eine Bewegung entlang der Längsachse der Herzklappenprothese. Durch die erfinderische Verkleinerung der von den mindestens zwei Bauteilen gebildeten und umschlossenen rotationssymmetrischen Räume mittels einer Bewegung der Bauteile aufeinander zu und ineinander entlang der Längsachse des Implantats wird das Risiko der Schädigung der Herzklappenprothese deutlich minimiert.

Unter einem vom Bauteil umschlossenen Raum wird im Rahmen dieser Anmeldung jeder Raum verstanden, den das Bauteil durch seine dreidimensionale Ausformung umschließt. Dies können beispielsweise Hohlräume in einem Vollmaterialkörper oder Räume sein, die durch mehrere hervorstehende Ausformungen des Bauteils gebildet werden. So würde beispielsweise ein rohrförmiger Körper bestehend aus einer Gitter- oder Stabstruktur in seinen Inneren einen zylindrischen Raum umschließen. Analog zu einem annähernd zylindrischen oder kegelstumpfförmigen Raum, den ein Wassereimer in seinem Inneren umschließt.

Unter einem rotationssymmetrischen umschlossenen Raum wird entsprechend im Rahmen dieser Anmeldung ein umschlossener Raum verstanden, der eine Symmetrieachse aufweist, um die herum der umschlossene Raum gedreht werden kann, ohne seine Form zu ändern. Im Beispiel des rohrförmigen Körpers ist dies die Zylinderachse, im Beispiel des Wassereimers die Zentralachse des Zylinders oder des Kegelstumpfes.

Die Bauteile der vorliegenden Erfindung umschließen jeweils zumindest teilweise einen derartigen rotationssymmetrischen Raum, welcher sich in einer Richtung entlang der Symmetrieachse bzw. Rotationsachse verjüngt bzw. verkleinert.

Die zumindest zwei Bauteile werden nach dem erfindungsgemäßen Verfahren entsprechend derart aufeinander zu und ineinander bewegt, dass sich der von ihnen gemeinsam umschlossene Raum verkleinert. Die Herzklappenprothese befindet sich bei dieser Bewegung in dem von beiden Bauteilen gemeinsam umschlossenen Raum und die Bewegung der beiden Bauteile erfolgt entlang der Längsachse der Herzklappenprothese. Dabei liegt die Längsachse der Herzklappenprothese auf der Symmetrieachse, der von den beiden Bauteilen umschlossenen rotationssymmetrischen Räume (Längsachse der Implantat und Symmetrieachse des rotationssymmetrischen Raumes fallen zusammen).

Unter der Längsachse der Herzklappenprothese wird im Rahmen dieser Anmeldung die zentrale Achse des Implantats verstanden. Die Längsachse der Herzklappenprothese stimmt entsprechend mit der Katheterachse des Katheters überein, auf den die Herzklappenprothese angebracht wird. Bei einer Herzklappenprothese, die im Wesentlichen die Form eines Röhrchens hat, ist die Längsachse entsprechend die Zylinderachse.

Unter Katheterschaft wird im Rahmen dieser Anmeldung zumindest der Teil des Katheters verstanden, auf den die Herzklappenprothese angebracht wird.

Die Herzklappenprothese ist eine künstliche Herzklappe mit einem selbstexpandierenden Grundkörper. Bei einer Herzklappenprothese sollen die Klappen öffnen und schließen, um einen Blutstrom zu ermöglichen oder zu stoppen. Der Blutstrom fließt dabei nach Implantation der Herzklappenprothese entsprechend entlang der Längsachse der Herzklappenprothese. Entsprechend klappen die Herzklappen der Herzklappenprothese auf diese Längsachse zu bzw. von ihr weg. Die Längsachse der Herzklappenprothese wird durch die Zentralachse des Grundkörpers gebildet, der die Klappen trägt. Durch das Anbringen der Herzklappenprothese auf den Katheterschaft mittels einer Bewegung der zumindest zwei Bauteile aufeinander zu und ineinander entlang dieser Längsachse der Herzklappenprothese wird das Risiko des Abknickens oder Verkantens der Klappen deutlich minimiert. Die Bewegung der Bauteile aufeinander zu und ineinander entlang der Längsachse entspricht der möglichen Bewegung der Klappen, die auf die Längsachse hin beziehungsweise von ihr weg klappen. Entsprechend wird das Risiko minimiert, weil nach dem erfindungsgemäßen Verfahren die Bewegung der Bauteile beim Anbringen der Bewegung der Herzklappen in Funktion entspricht.

Vorteilhafterweise ist der rotationssymmetrische und sich entlang der Symmetrieachse verjüngende Raum ein Konus. Die Verwendung eines konusförmigen umschlossenen Raumes ist besonders vorteilhaft, weil so in einfacher Art eine stetige und kantenfreie Verjüngung des umschlossenen Raumes gewährleistet wird. Prinzipiell sind jedoch auch andere sich verjüngenden rotationssymmetrische Formen wie eine Pyramide oder ähnliches denkbar.

Unter einem Konus wird im Rahmen der Anmeldung ein Kegel oder ein Kegelstumpf verstanden. Bei einem Kegelstumpf wird entsprechend unter der Spitze des Konus die kleinere Fläche verstanden.

Bevorzugt bewegen sich die konischen Räume derart ineinander, dass der von zumindest zwei Bauteilen umschlossene Raum immer kleiner wird. In dieser Ausgestaltung der Erfindung werden zumindest zwei Bauteile derart ineinander geschoben, dass der von den beiden Konen umschlossene Raum immer kleiner wird. Zweckmäßigerweise sind die Spitzen der beiden Konen entgegengesetzt orientiert und liegen auf der Längsachse Herzklappenprothese. Das Ineinanderschieben der Konen reduziert so kontinuierlich den Durchmesser des freien Raumes um die Längsachse der Herzklappenprothese, der von den Konen der zumindest zwei Bauteile umschlossen wird.

Erfindungsgemäß wird entlang der Längsachse der Herzklappenprothese zumindest während der Bewegung der mindestens zwei Bauteile aufeinander zu und ineinander ein Fluidstrom, insbesondere ein Volumenstrom, angelegt. Der Fluidstrom wird entlang der Längsachse während des Anbringens angelegt. Dieser Fluidstrom sorgt für eine Vororientierung der Herzklappen, wodurch das Risiko des Abknicken oder Verkantens noch weiter minimiert wird.

Alternativ oder zusätzlich weist erfindungsgemäß mindestens ein Bauteil einen Dorn auf, der zentral derart in dem vom Bauteil umschlossenen Raum orientiert ist, dass er während der Bewegung der mindestens zwei Bauteile aufeinander zu und ineinander auf der Längsachse der Herzklappenprothese liegt. Der Dorn sorgt für eine Vororientierung der Herzklappen. Die Bauteile werden in dieser Ausgestaltung der Erfindung derart ineinander geführt, dass der Dorn in Öffnungsrichtung in die Herzklappen der Herzklappenprothese geführt wird. Die Herzklappen gehen so sauber auf und können sich nach Anbringen der Prothese auf den Katheterschaft an diesen anlegen.

Vorrichtungsseitig wird die gestellte Aufgabe von einem System zum Anbringen einer Herzklappenprothese auf einem Katheterschaft gelöst, welches mindestens zwei Bauteile umfasst, die jeweils mindestens teilweise einen rotationssymmetrische und sich entlang der Symmetrieachse verjüngende Raum umschließen, und wobei zumindest eines der Bauteile an dem verjüngten Ende des umschlossenen rotationssymmetrischen Raumes eine Öffnung aufweist, die geeignet ist, vom Katheterschaft passiert zu werden und mindestens ein Bauteil einen Dorn aufweist, der zentral in dem vom Bauteil umschlossenen Raum orientiert ist und auf der Symmetrieachse des rotationssymmetrischen Raumes liegt.

Das erfindungsgemäße System mit einer Öffnung an dem verjüngten Ende des umschlossenen rotationssymmetrischen Raumes erlaubt die Durchführung des erfindungsgemäßen Verfahrens. Der Katheterschaft kann die Öffnung am verjüngten Ende des rotationssymmetrischen Raumes passieren. Dadurch wird die Bewegung der zumindest zwei Bauteile aufeinander zu und ineinander entlang der Längsachse der Herzklappenprothese erlaubt. Im angebrachten Zustand auf dem Katheterschaft liegen Längsachse der Herzklappenprothese und Katheterachse aufeinander. Durch diese Öffnung kann auch der Katheterschaft mit angebrachter Herzklappenprothese wieder aus dem erfinderischen System entfernt werden.

Zweckmäßigerweise ist der rotationssymmetrische und sich entlang der Symmetrieachse in einer Richtung verjüngende Raum ein Konus. Bevorzugt umschließt mindestens ein oder zwei Bauteile einen Raum, der von einem Konus und einem Zylinder gebildet wird. Ein Konus, optional in Kombination mit einem Zylinder, ist die einfachste räumliche Geometrie zur Verwirklichung der Erfindung. Bauteile die derartige Räume umschließen sind besonders einfach zu fertigen. Je nach konkreter Form der anzubringenden Herzklappenprothese sind jedoch andere Formen im Rahmen der Erfindung denkbar.

Besonders bevorzugt weist jedes der mindestens zwei Bauteile Aussparungen auf, in die das/die jeweils andere/n Bauteil/e hineingreifen können. In dieser Ausgestaltung der Erfindung besteht das System aus mindestens zwei, bevorzugt zwei, Bauteilen. Dabei weist mindestens eines dieser Bauteile Aussparungen auf, die von Ausformungen des/der anderen Bauteils/Bauteile passgenau gefüllt werden können. Durch diese Aussparungen und entsprechende komplementären Ausformungen können die Bauteile einfach und geführt aufeinander zu und ineinander bewegt werden. Bevorzugt weist dabei jedes Bauteil sowohl Aussparungen als auch Ausformungen auf, die jeweils mit einem anderen Bauteil korrespondieren. Durch diese genannten Ausgestaltungen wird sichergestellt, dass Herzklappenprothesen immer auf die gleiche Weise auf gleichen Katheterschäften angebracht werden. Zusätzlich kann durch die Ausformungen und entsprechenden Aussparungen sichergestellt werden, dass die Symmetrieachsen der Bauteile und die Längsachse der Herzklappenprothese übereinander liegen.

Vorteilhafterweise weist ein Bauteil einen Anschlag für den Katheterschaft auf und/oder ein Bauteil eine Aufnahme für die Herzklappenprothese auf. Dabei können Anschlag und Aufnahme im gleichen oder in zwei verschiedenen Bauteilen sein. Hierdurch wird ebenfalls die Reproduzierbarkeit verbessert und die Handhabung des erfindungsgemäßen Systems vereinfacht.

Erfindungsgemäß weist mindestens ein Bauteil einen Dorn auf, der zentral in dem vom Bauteil umschlossenen Raum orientiert ist und auf der Symmetrieachse des rotationssymmetrischen Raumes liegt. Dieser Dorn sorgt wie bereits verfahrensseitig beschrieben für eine Ausrichtung der Herzklappen.

Die vorliegende Erfindung stellt ein einfaches Verfahren und System zur Verfügung, um eine Herzklappenprothese auf einen Katheterschaft anzubringen. Das System ist einfach zu handhaben, die Ausführung des Verfahrens erfordert kaum Vorkenntnisse. Das erfindungsgemäße Verfahren und das erfindungsgemäße System eignen sich zum sicheren Anbringen von Herzklappenprothesen auf einem Katheterschaft. Die Gefahr des Abknickens oder Verkantens der Klappen beim Anbringen der Herzklappenprothese auf dem Katheterschaft wird deutlich minimiert. Dadurch erhöht sich Lebensdauer und Funktionalität des Implantats.

Im Folgenden soll die Erfindung anhand des in den Figuren dargestellten Ausführungsbeispieles der Erfindung näher erläutert werden.

Es zeigen:
- Figur 1: ein Ausführungsbeispiel des erfindungsgemäßen Systems mit zwei Bauteilen, wobei beide Bauteile im getrennten Zustand dargestellt sind,
- Figur 2: das Ausführungsbeispiel aus Figur 1 in dem Zustand, in dem beide Bauteil komplett ineinander geschoben sind, und
- Figuren 3a-c: eine Ausführungsform des erfindungsgemäßen Verfahrens mit dem in Figur 1 und 2 dargestellten Ausführungsbeispiel des erfindungsgemäßen Systems

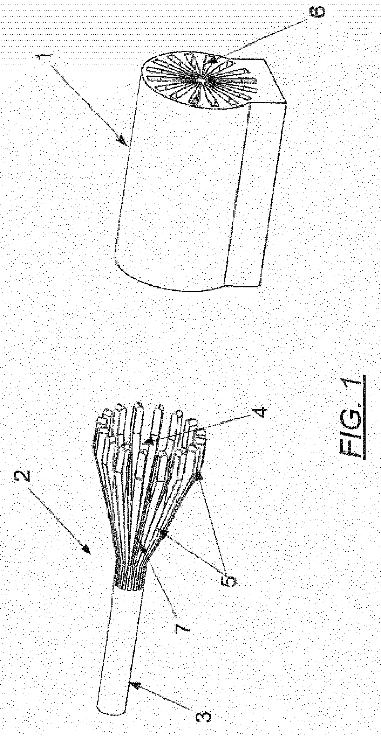

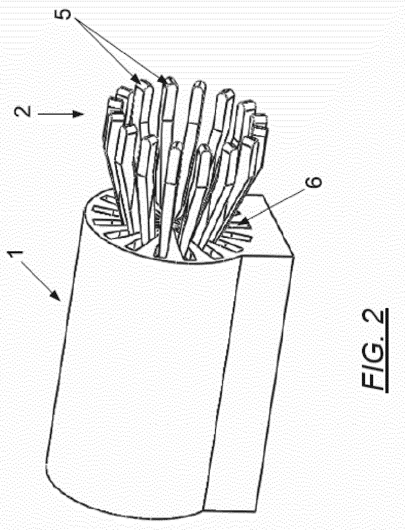

In den Figuren wird ein Ausführungsbeispiel des erfindungsgemäßen Systems dargestellt, welches aus zwei Bauteilen besteht. Figur 1 zeigt die beiden Bauteile 1 und 2 im getrennten Zustand, während in Figur 2 die beiden Bauteile 1 und 2 ineinander bewegt wurden.

Bauteil 1 ist zylindrisch und umschließt im Inneren einen Raum, welcher sich aus einem Zylinder und einem Konus zusammensetzt (nicht dargestellt in Fig. 1-2). Bauteil 2 besteht aus einem Handgriff 3 und Ausformungen 5. Von den Ausformungen 5 des Bauteils 2 wird ein Raum umschlossen, der sich aus einem Zylinder 4 und einen Konus 7 zusammensetzt. Bauteil 1 weist Aussparungen 6 auf, die derart ausgeformt sind, dass sie die Ausformungen 5 von Bauteil 2 aufnehmen können.

Wie in Figur 2 dargestellt können die Bauteile 1 und 2 aufeinander und ineinander bewegt werden, wobei durch die Aussparungen 6 in Bauteil 1 und die entsprechend geformten Ausformungen 5 von Bauteil 2 die Bewegung der Bauteile 1 und 2 ineinander geführt abläuft.

Sowohl der von Bauteil 2 umschlossene zylindrische Raum 4 als auch der konische Raum 7 sind rotationssymmetrisch. Die Symmetrieachse verläuft durch die Zentralachse des Handgriffes 3. Der von Bauteil 1 umschlossene zylindrische Raum und der von Bauteil 1 umschlossene konische Raum (nicht dargestellt in Fig. 1-2) sind ebenfalls rotationssymmetrisch wobei die Symmetrieachse hier auf die zentrale Achse des zylindrischen Bauteils 1 fällt.

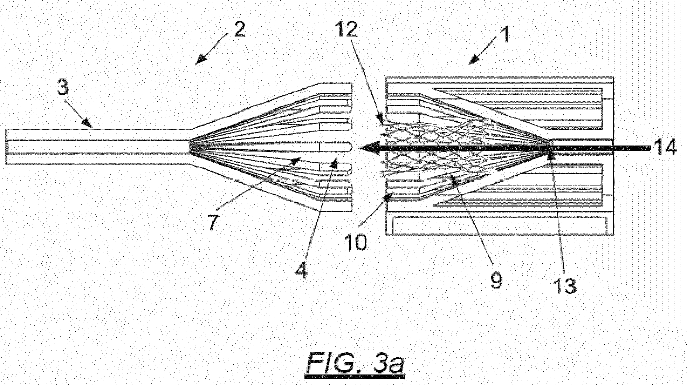

In den Figuren 3a bis 3c sind die beiden Bauteile 1, 2 aus den Figuren 1 und 2 jeweils im Schnitt entlang der Symmetrieachse dargestellt. Anhand der Figuren 3a-3c wird im Folgenden ein Ausführungsbeispiel des erfindungsgemäßen Verfahrens zum Anbringen einer Herzklappenprothese 12 auf einem Katheterschaft (nicht dargestellt) beschrieben.

Figur 3a zeigt die Ausgangssituation. Die Herzklappenprothese 12 wird in Bauteil 1 positioniert. Den von Bauteil 1 umschlossenen zylindrischen Raum 10 schließt sich unmittelbar ein konischer Raum 9 an. An der Spitze des Konus 9 ist eine durchgehende Öffnung 13, die bis aus Bauteil 1 hinaus reicht. Durch diese Öffnung 13 wird der Katheterschaft entlang des Pfeiles 14 in das Bauteil 1 geschoben.

Zu Beginn des Verfahrens werden Bauteil 1 und Bauteil 2 entlang der Längsachse der Herzklappenprothese 12 aufeinander zu bewegt. Die Bauteile 1, 2 werden dabei so orientiert und aufeinander zu bewegt, das Längsachse der Herzklappenprothese 12, Längsachse der Bauteile 1,2, die Symmetrieachsen der von den Bauteilen 1, 2 umschlossenen konischen Räumen 7, 9 und zylindrischen Räumen 4, 10 und die Katheterachse aufeinander fallen und entlang dem Pfeil 14 orientiert sind.

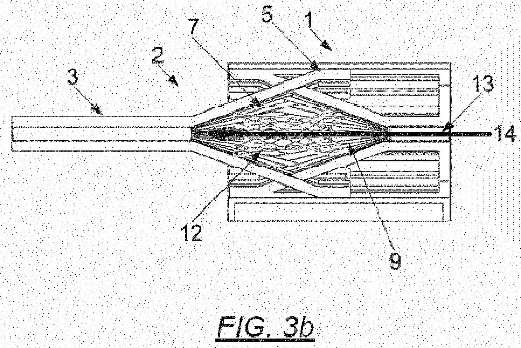

Figur 3b zeigt ein Verfahrensstadium in dem die beiden Bauteile 1 und 2 schon ineinander bewegt wurden. Zu den in Figur 3b gezeigten Zeitpunkt hat sich der von beiden Bauteilen gemeinsam umschlossene Raum schon verkleinert. Als die beiden Bauteile 1 und 2 sich in Figur 3a aufeinander zu bewegten, hat der von beiden Bauteilen 1 und 2 gemeinsam umschlossene Raum den zylindrischen Teil 4 und den konischen Teil 7 von Bauteil 2 sowie den zylindrischen Teil 10 und den konischen Teil 9 von Bauteil 1 umfasst. Im Verfahrensstadium von Figur 3b besteht der von beiden Bauteilen 1, 2 gemeinsam umschlossene Raum nur noch aus den beiden konischen Teile 7 und 9.

Der Katheterschaft wurde in Figur 3b entlang des Pfeiles 14 in Bauteil 2 vorgeschoben. Der Durchmesser der Herzklappenprothese 12 hat sich in Figur 3b bereits verkleinert.

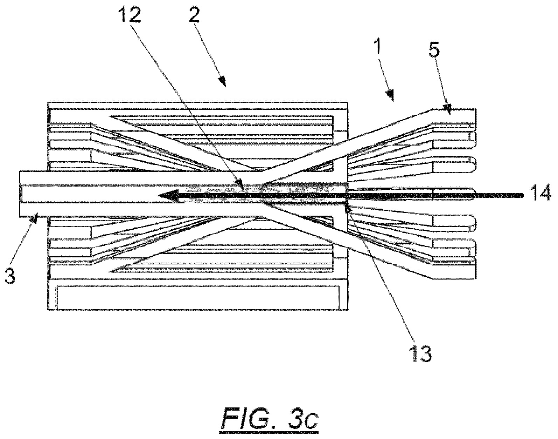

Wie in Figur 3c sichtbar wird der von beiden Bauteilen gemeinsam umschlossene Raum 7, 9 immer weiter verkleinert bis die Herzklappenprothese 12 auf den Katheterschaft gepresst und entsprechend angebracht ist. Die beiden Bauteile 1 und 2 werden dabei bis zu einem Anschlag (am Schaft 3, nicht dargestellt) ineinander geschoben. Der Katheterschaft mit der darauf angebrachten Herzklappenprothese 12 wird anschließend entgegengesetzt dem Pfeil 14 aus dem System gezogen. Beim Herausziehen des Katheterschafts wird die Herzklappenprothese auf dem Schaft mit einem Schlauch gesichert, der synchron über die Herzklappenprothese geschoben wird.

Das System und das erfindungsgemäße Verfahren führen zu Bewegungen in Richtung 14 der Längsachse der Herzklappenprothese 12. Dadurch wird ein Anschmiegen der Klappen an den Katheterschaft erreicht und die Gefahren des Verkantens oder Verknickens der Klappen minimiert.

In einer Ausgestaltung dieses Ausführungsbeispiels kann über die Öffnung 13 oder den Schaft des Handgriffes 3 noch zusätzlich ein Volumenstrom eingeleitet werden, der für eine Vororientierung der Herzklappen in die Offenstellung sorgt. Die Zufuhr des Volumenstroms hängt dabei von der Orientierung der Herzklappenprothese 12 auf dem Katheter ab.

## Patentansprüche

1. Verfahren zum Anbringen einer künstlichen Herzklappe (12) mit einem selbstexpandierenden Grundkörper, kurz: Herzklappenprothese, auf einem Katheterschaft, wobei die Herzklappenprothese (12) im Durchmesser durch eine Relativbewegung von mindestens zwei Bauteilen (1,2), die jeweils zumindest teilweise einen rotationssymmetrischen und sich entlang dessen Symmetrieachse in einer Richtung verjüngenden Raum (7,9) umschließen, reduziert wird,
wobei die Relativbewegung der mindestens zwei Bauteile (1,2) entlang der Längsachse der Herzklappenprothese aufeinander zu und derart ineinander erfolgt, dass sich der von den mindestens zwei Bauteilen (1,2) gemeinsam umschlossene Raum (7,9) verkleinert,
wobei sich die Herzklappenprothese (12) in dem von den mindestens zwei Bauteilen (1,2) gemeinsam umschlossenen Raum befindet und derart orientiert ist, dass die Längsachse der Herzklappenprothese und die Symmetrieachsen (14) der rotationssymmetrischen Räume aufeinander liegen,
**dadurch gekennzeichnet, dass**
mindestens ein Bauteil (1, 2) einen Dorn aufweist, der zentral derart in dem vom Bauteil (1, 2) umschlossenen Raum (4, 7, 10, 9) orientiert ist, dass er während der Bewegung der mindestens zwei Bauteile aufeinander zu und ineinander auf der Längsachse der Herzklappenprothese liegt, und/oder
entlang der Längsachse der Herzklappenprothese (12) zumindest während der Bewegung der mindestens zwei Bauteile (1,2) aufeinander zu und ineinander ein Fluidstrom, insbesondere ein Volumenstrom, angelegt wird.

2. Verfahren nach Anspruch 1, **dadurch gekennzeichnet, dass** der Dorn und/oder der Fluidstrom für eine Vororientierung der Herzklappen sorgen.

3. Verfahren nach Anspruch 1 oder 2, **dadurch gekennzeichnet, dass** der rotationssymmetrische und sich entlang der Symmetrieachse verjüngende Raum ein Konus (7,9) ist.

4. Verfahren nach Anspruch 3, **dadurch gekennzeichnet, dass** sich die konischen Räume (7,9) derart ineinander bewegen, dass der von den zumindest zwei Bauteilen (1,2) umschlossene Raum immer kleiner wird.

5. System zum Anbringen einer Herzklappenprothese (12) auf einem Katheterschaft umfassend mindestens zwei Bauteile (1,2), die jeweils mindestens teilweise einen rotationssymmetrische und sich entlang der Symmetrieachse verjüngende Raum (7, 9) umschließen,
wobei zumindest eines der Bauteile (1,2) an dem verjüngten Ende des umschlossenen rotationssymmetrischen Raumes eine Öffnung (13) aufweist, die geeignet ist, vom Katheterschaft passiert zu werden, wobei die mindestens zwei Bauteile (1,2) derart ausgestaltet sind, dass eine Relativbewegung der mindestens zwei Bauteile (1,2) entlang der Längsachse der anzubringenden Herzklappenprothese aufeinander zu und derart ineinander erfolgen kann, dass sich der von den mindestens zwei Bauteilen (1,2) gemeinsam umschlossene Raum (7,9) verkleinert,
**dadurch gekennzeichnet, dass**
mindestens ein Bauteil (1,2) einen Dorn aufweist, der zentral in dem vom Bauteil umschlossenen Raum orientiert ist und auf der Symmetrieachse (14) des rotationssymmetrischen Raumes liegt.

6. System nach Anspruch 5, **dadurch gekennzeichnet, dass** der rotationssymmetrische und sich entlang der Symmetrieachse in einer Richtung verjüngende Raum ein Konus (7, 9) ist.

7. System nach Anspruch 6, **dadurch gekennzeichnet, dass** mindestens ein oder zwei Bauteile (1,2) einen Raum umschließen, der von einem Konus (7, 9) und einem Zylinder (4, 10) gebildet wird.

8. System nach einem der Ansprüche 5 bis 7, **dadurch gekennzeichnet, dass** jedes der mindestens zwei Bauteile (1,2) Aussparungen (6) aufweist, in die das/die jeweils andere/n Bauteil/e hineingreifen können.

9. System nach einem der Ansprüche 5 bis 8, **dadurch gekennzeichnet, dass** mindestens ein Bauteil (1,2) einen Anschlag (8) für den Katheterschaft aufweist.

10. System nach einem der Ansprüche 5 bis 9, **dadurch gekennzeichnet, dass** mindestens ein Bauteil (1,2) eine Aufnahme (11) für die Herzklappenprothese aufweist.

## Claims

1. A method for mounting an artificial heart valve (12) having a self-expanding base body, hereinafter: heart valve prosthesis, on a catheter shaft, the diameter of the heart valve prosthesis (12) being reduced by a relative movement of at least two components (1, 2), which each at least partially enclose a rotation-symmetrical space (7, 9) tapering along the axis of symmetry thereof in one direction,
the relative movement of the at least two components (1, 2) taking place toward one another and into one another along the longitudinal axis of the heart valve prosthesis in such a way that the space (7, 9) enclosed jointly by the at least two components (1, 2) is decreased,
the heart valve prosthesis (12) being located in the space enclosed jointly by the at least two components (1, 2) and being oriented in such a way that the longitudinal axis of the heart valve prosthesis and the axes of symmetry (14) of the rotation-symmetrical spaces are located on top of one another,
**characterized in that**
at least one component (1, 2) comprises a mandrel, which is oriented centrally in the space (4, 7, 10, 9) enclosed by the component (1, 2) so as to be located on the longitudinal axis of the heart valve prosthesis during the movement of the at least two components toward one another and into one another, and/or
a fluid flow, and in particular a volume flow, is applied along the longitudinal axis of the heart valve prosthesis (12) at least during the movement of the at least two components (1, 2) toward one another and into one another.

2. The method according to claim 1, **characterized in that** the mandrel and/or the fluid flow ensure a pre-orientation of the heart valves.

3. The method according to claim 1 or 2, **characterized in that** the rotation-symmetrical space tapering along the axis of symmetry is a cone (7, 9).

4. The method according to claim 3, **characterized in that** the conical spaces (7, 9) move into one another in such a way that the space enclosed by at least two components (1, 2) becomes increasingly smaller.

5. A system for mounting a heart valve prosthesis (12) on a catheter shaft, comprising at least two components (1, 2) which each, at least partially, enclose a rotation-symmetrical space (7, 9) tapering along the axis of symmetry,
at least one of the components (1, 2), at the tapered end of the enclosed rotation-symmetrical space, having an opening (13) that is suitable for the catheter shaft to be passed through,
the at least two components (1, 2) being designed in such a way that a relative movement of the at least two components (1, 2) along the longitudinal axis of the heart valve prosthesis to be mounted can take place toward one another and into one another in such a way that the space (7, 9) enclosed jointly by the at least two components (1, 2) is decreased,
**characterized in that**
at least one component (1, 2) comprises a mandrel, which is oriented centrally in the space enclosed by the component and is located on the axis of symmetry (14) of the rotation-symmetrical space.

6. The system according to claim 5, **characterized in that** the rotation-symmetrical space tapering along the axis of symmetry in one direction is a cone (7, 9).

7. The system according to claim 6, **characterized in that** at least one or two components (1, 2) enclose a space formed by a cone (7, 9) and a cylinder (4, 10).

8. The system according to any one of claims 5 to 7, **characterized in that** each of the at least two components (1, 2) has recesses (6) in which the respective other component(s) can engage.

9. The system according to any one of claims 5 to 8, **characterized in that** at least one component (1, 2) comprises a stop (8) for the catheter shaft.

10. The system according to any one of claims 5 to 9, **characterized in that** at least one component (1, 2) comprises a seat (11) for the heart valve prosthesis.

## Revendications

1. Procédé de fixation d'une valve cardiaque (12) synthétique avec un corps de base auto expansif, en résumé, une prothèse de valve cardiaque, sur une tige de cathéter, où la prothèse de valve cardiaque (12) est réduite dans le diamètre par un mouvement relatif d'au moins deux composantes (1, 2) qui entourent respectivement au moins partiellement un espace (7, 9) symétrique en rotation et s'amenuisant dans une direction le long de son axe de symétrie,
où le mouvement relatif des au moins deux composantes (1, 2) s'effectue le long de l'axe longitudinal de la prothèse de valve cardiaque de l'une vers l'autre et de l'une dans l'autre de telle manière que l'espace (7, 9) entouré par les au moins deux composantes ensemble se rétrécit,
où la prothèse de valve cardiaque (12) se trouve dans l'espace entouré par les au moins deux composantes et est orienté de telle manière que l'axe longitudinal de la prothèse de valve cardiaque et les axes de symétrie (4) des espaces symétriques en rotation sont l'un au dessus de l'autre,
**caractérisé en ce**
**qu'**au moins une composante (1, 2) présente une broche qui est orientée de manière centrale de telle façon dans l'espace (4, 7, 10, 9) entouré par la composante (1, 2) qu'elle se situe sur l'axe longitudinal de la prothèse de valve cardiaque pendant le mouvement des au moins deux composantes l'une vers l'autre et de l'une dans l'autre, et/ou
**qu'**un flux de fluide, notamment un flux volumique, est appliqué le long de l'axe longitudinal de la prothèse de valve cardiaque (12) au moins pendant le mouvement des au moins deux composantes (1, 2) l'une vers l'autre et de l'une dans l'autre.

2. Procédé selon la revendication 1, **caractérisé en ce que** la broche et/ou le flux de fluide oeuvrent en faveur d'une pré-orientation des valves cardiaques.

3. Procédé selon la revendication 1 ou 2, **caractérisé en ce que** l'espace symétrique en rotation et s'amenuisant le long de l'axe de symétrie est un cône (7, 9).

4. Procédé selon la revendication 3, **caractérisé en ce que** les espaces coniques (7, 9) se déplacent l'un dans l'autre de telle manière que l'espace entouré par les au moins deux composantes (1, 2) devient toujours plus petit.

5. Système destiné à la fixation d'une prothèse de valve cardiaque (12) sur une tige de cathéter comprenant au moins deux composantes (1, 2) qui entourent respectivement au moins partiellement un espace (7, 9) symétrique en rotation et s'amenuisant le long de son axe de symétrie,
où au moins une des composantes (1, 2) présente une ouverture (13) à l'extrémité rétrécie de l'espace entouré symétrique en rotation qui est appropriée pour être traversée par la tige de cathéter,
où les au moins deux composantes (1, 2) sont conçues de telle manière qu'un mouvement relatif des au moins deux composantes (1, 2) le long de l'axe longitudinal de la prothèse de valve cardiaque à fixer l'une sur l'autre et l'une vers l'autre peut s'effectuer de telle manière que l'espace (7, 9) entouré par les au moins deux composantes (1, 2) ensemble se rétrécit,
**caractérisé en ce**
**qu'**au moins une composante (1, 2) présente une broche qui est orientée de manière centrale dans l'espace entouré par la composante qui se situe sur l'axe de symétrie (14) de l'espace symétrique en rotation.

6. Système selon la revendication 5, **caractérisé en ce que** l'espace symétrique en rotation et s'amenuisant le long de l'axe de symétrie dans une direction est un cône (7, 9).

7. Système selon la revendication 6, **caractérisé en ce qu'**au moins une ou deux composantes (1, 2) entourent un espace qui est formé par un cône (7, 9) et un cylindre (4, 10).

8. Système selon l'une des revendications5 à 7, **caractérisé en ce que** chacune des au moins deux composantes (1, 2) présente des évidements (6), dans lesquels l'autre/les autres composante/s peut/peuvent pénétrer en prise.

9. Système selon l'une des revendications 5 à 8, **caractérisé en ce qu'**au moins une composante (1, 2) présente une butée (8) pour la tige de cathéter.

10. Système selon l'une des revendications 5 à 9, **caractérisé en ce qu'**au moins une composante (1, 2) présente un logement (11) pour la prothèse de valve cardiaque.
